# EUROPÄISCHE PATENTANMELDUNG

(11) **EP 0 816 845 A1**
(43) Veröffentlichungstag der Anmeldung: **07.01.1998**
(21) Anmeldenummer: 97110831.1
(22) Anmeldetag: 01.07.1997
(51) Int. Cl.: G01N 33/00

(54) **Verfahren zum Herstellen eines Gasgemisches**

(30) Priorität: 03.07.1996 DE 19626749
(71) Anmelder: Linde Aktiengesellschaft, 65189 Wiesbaden (DE)
(72) Erfinder: Weis, Volker, 85276 Pfaffenhofen (DE); Gaier, Hans, Dr., 85276 Pfaffenhofen (DE); Wunschel, Hans-Jörg, Dr., 85716 Unterschleissheim (DE)
(74) Vertreter: Kasseckert, Rainer

(57) **Zusammenfassung**

Verfahren zum Herstellen eines Gasgemisches aus mindestens einer verdampfbaren Substanz, insbesondere eines Prüfgases, in einem Druckgasbehälter durch
- thermolytisches Spalten einer verdampfbaren Substanz in einem Reaktor (Kammer 2)
- Überleiten der so erhaltenen verdampften Substanz in einen getrennten Reaktor (Kammer 4)
- Mischen mit einem Restgas (Gas oder Gasgemisch aus Gasen, die nicht mit der verdampften Substanz reagieren, insbesondere Edelgas oder Stickstoff) in dem zweiten Reaktor (Kammer 4)
- Spülen mit Restgas in den Druckgasbehälter.
Verwendbar ist das Verfahren insbesondere zur Herstellung eines Gasgemisches, das monomeres Formaldehyd enthält.

## Beschreibung

Die Erfindung betrifft ein Verfahren zum Herstellen eines Gasgemisches aus mindestens einer verdampfbaren Substanz, insbesondere eines Prüfgasgemisches.

Insbesondere in der Automobilindustrie sind derzeit Prüfgase erwünscht, die einen definierten Gehalt an monomerem Formaldehyd haben. Dies dient zur Kalibrierung von Meßsystemen oder zu Vergleichsmessungen des Formaldehydgehaltes von Auspuffgasen. Solche Gase - in denen Formaldehyd in monomerer Form vorliegt, die über lange Zeit lagerfähig sind und die in ihrer Zusammensetzung auch nach langer Lagerung nicht wesentlich verändert sind - sind bisher auf dem Markt nicht erhältlich.

In der Patentliteratur beschäftigen sich einige Dokumente mit der Herstellung von reinem gasförmigen Formaldehyd.

Die DE-AS 1 253 259 beschreibt ein Verfahren zur Herstellung von reinem gasförmigen Formaldehyd durch Absorption von Formaldehyd aus Gasen der Formaldehydsynthese mit einem mehrwertigen Alkohol, chemische Zersetzung des gebildeten Halbformals und Rückführung des gewonnenen mehrwertigen Alkohols in die Absorption in einem mehrstufigen Prozeß.

Die DE-AS 1 283 218 beschreibt ein Verfahren zur Herstellung von sehr reinem Formaldehyd durch thermische Depolymerisation von niedermolekularem Polyoximethylen mit geringem Wassergehalt, welches vorher einer Wärmebehandlung in einem geschlossenen System unterworfen und anschließend getrocknet wurde.

Die DE-OS 28 15 314 beschreibt ein Verfahren zum Reinigen gasförmigen Formaldehyds mit Hilfe von Wirbelschichten und festen Absorptionsmitteln.

Die DE-AS 1 232 565 beschreibt ein Verfahren zur Herstellung von sehr reinem monomerem Formaldehyd durch thermische Depolymerisation von Trioxan, bei dem die Depolymerisation in einem bestimmten Temperaturbereich und in einem Strom eines indifferenten, trockenen und reinen Gases durchgeführt wird.

Alle bekannten Verfahren führten bisher nicht zu Gasen, die in Druckgasbehältern über längere Zeit aufbewahrt werden können und ihren Gehalt an monomerem Formaldehyd konstant behielten.

Aufgabe der Erfindung ist es daher, ein Verfahren zur Herstellung von Gasgemischen aus verdampfbaren Substanzen, insbesondere eines lagerbeständigen Formaldehyd-Gasgemisches, in einem Druckgasbehälter anzugeben.

Diese Aufgabe wird erfindungsgemäß gelöst von dem Verfahren des Anspruchs 1. Ausgestaltungen des Verfahrens, Verwendungen, und das Gas selbst sind Gegenstände von Unteransprüchen.

Die Besonderheit des Verfahrens liegt darin, daß mindestens zwei Reaktionskammern verwendet werden, wobei in der ersten Kammer ein thermolytisches Spalten der Ausgangsprodukte stattfindet, diese Produkte dann in die zweite Kammer geführt werden, in welcher eine Mischung mit einem Restgas erfolgt. Dieses Gas wird dann in die Druckgasbehälter abgefüllt. Mit dem erfindungsgemäßen Verfahren gelang es erstmals, ein langzeitstabiles Gasgemisch mit einem gewünschten Gehalt an monomerem Formaldehyd herzustellen.

Die thermolytische Spaltung der Ausgangsprodukte, Paraformaldehyd, Trioxan oder Alpha-Polyoximethylen erfolgt in einem Bereich zwischen 30 bis 400°C, bevorzugt zwischen 50 und 200°C.

Die Mischung des monomeren Formaldehyd erfolgt in der oberen Reaktorkammer. Die Überführung des Monomers kann aufgrund der Druckdifferenz der beiden Kammern verlaufen, wobei die obere Reaktorkammer einen geringeren Druck aufweist. Das signifikante Merkmal hierbei ist die Druckdifferenz zwischen beiden Reaktoren, welche sich nicht in direkten Zahlen ausdrücken läßt. Die Überführung kann auch durch Einleiten von Restgas in den ersten Reaktor mit anschließender Überführung in den zweiten Reaktor erfolgen, wodurch dann gleichzeitig die erfindungsgemäße Mischung mit dem Restgas erfolgt. Bevorzugt wird das Restgas mittels einer zerstäubenden Düse in den das Formaldehyd enthaltenden zweiten Reaktor eingeführt. Als günstige Zeit für die Mischung oder die Stabilisierung des Gemisches haben sich eine Minute bis ein Tag, bevorzugt eine Minute bis eine Stunde, erwiesen.

In einer bevorzugten Ausführung des Verfahrens wird der Ausgangsstoff im ersten Reaktor vorher durch Erwärmen, Kondensierenlassen und Spülen mit Restgas gereinigt. Die Reinigung des Ausgangsmaterials, z.B. Trioxan, kann z.B. durch Verdampfenlassen und anschließendes langsam wieder auskristallisieren lassen erfolgen. Anschließend wird der Behälter mit einem Edelgas oder Stickstoff belüftet und erneut evakuiert. Dieser Vorgang kann mehrmals wiederholt werden, wodurch eine sehr gute Reinigung des Trioxans erfolgt.

Das in der zweiten Reaktionskammer erhaltene Formaldehyd-Gasgemisch wird dann mit einem Restgas - meist der gleichen Zusammensetzung wie das erstverwendete - in einen Druckgasbehälter übergeführt. Dieses Gemisch kann nun als Vorgemisch für andere Prüfgase niedrigerer Konzentration dienen oder es kann an einem Füllstand auf einen beliebigen Enddruck (z.B. 150 bar oder 200 bar) aufgedrückt werden und so direkt als Prüfgas verwendet werden.

Mit dem erfindungsgemäßen Verfahren lassen sich Prüfgase herstellen, die Formaldehydanteile zwischen 0,01 ppm (V) bis 10%, insbesondere zwischen 0,01 ppm (V) bis 100 ppm (V) Formaldehyd oder 0,01 ppm (V) bis 50 % Formaldehyd oder 1 ppm (V) bis 5 % Formaldehyd enthalten, wobei der Rest Edelgase, Stickstoff oder nichtreaktive Gase sind. Ausgeschlossen sind insbesondere Sauerstoff, Wasserstoff oder Wasser enthaltende Gase.

Die Erfindung wird anhand zweier Figuren näher erläutert, die zwei Anordnungen zeigen, mit denen das erfindungsgemäße Verfahren durchführbar ist, insbesondere die Herstellung eines Prüfgases, das monomeres Formaldehyd enthält.

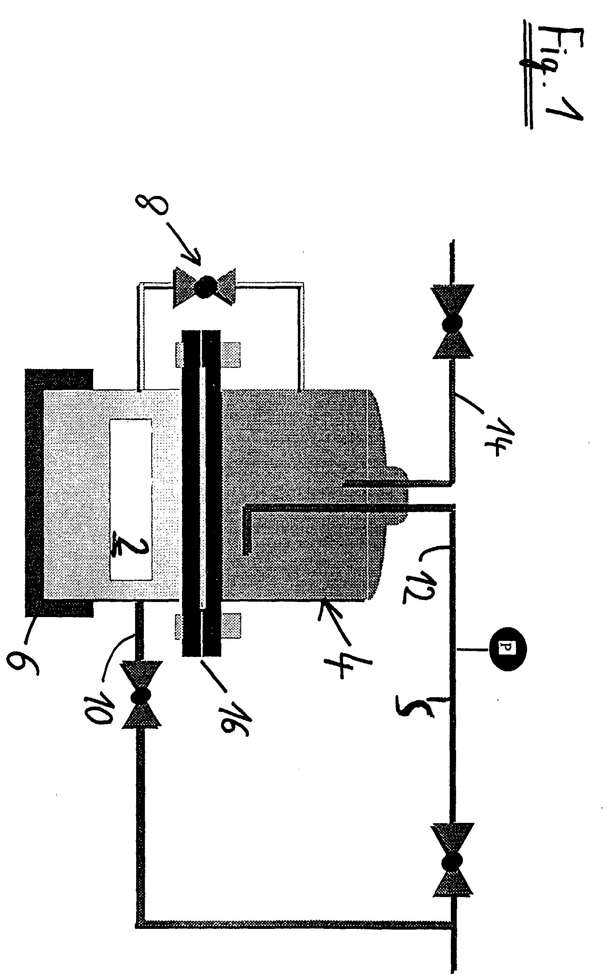

Figur 1 zeigt einen Zweikammerreaktor (Kammern 2, 4), wobei die untere Kammer 2 eine Heizung 6 aufweist. Zwischen beiden Kammern 2, 4 ist eine Bypassleitung 8 vorgesehen. In beide Kammern 2, 4 führen Anschlüsse 10, 12 für den Eintrag des Restgases. In Leitung 12 sind ein Druckmeßgerät P und ein Anschluß S für ein Sicherheitsventil gezeigt. In der oberen Kammer 4 ist ein Gasgemischausgang 14 vorgesehen. In diesen Zweikammerreaktor wird in die untere Kammer 2 Paraformaldehyd, Trioxan, Alpha-Polyoximethylen oder N-Hydroximethylbenzamid oder eine andere, Formaldehyd abgebende Substanz vorgelegt. In einem ersten Schritt kann diese Substanz getrocknet oder gereinigt werden, wenn ein besonders hochreines Formaldehyd-Gasgemisch gewünscht ist. Ansonsten oder anschließend erfolgt ein Beheizen der unteren Kammer 2 (Temperaturbereich 30 bis 400°C, bevorzugt 50 bis 150°C), wodurch die thermolytische Spaltung der Substanz in das monomere Formaldehyd erfolgt. Das so gewonnene Formaldehyd wird aufgrund der Druckdifferenz oder mit einem Edelgas oder Stickstoff (kein Reaktivgas) in die zweite Reaktorkammer 4, welche z.B. unter einem Unterdruck steht, überführt. Eine weitere Möglichkeit ist eine Überführung durch Umspülen des Gasvolumens aus Reaktorkammer 2 in Reaktorkammer 4 mit dem Restgas. Eine selbständige Überführung in die zweite obere Kammer 4 ist durch den Einbau einer Diffusionsmembran 16, welche beide Reaktorkammern 24 trennt, aufgrund des Konzentrationsgefälles, möglich. In der oberen Kammer wird es dann über einen bestimmten Zeitraum stabilisiert. Das so erhaltene Gemisch aus Restgas und monomerem Formaldehyd wird in einen Druckgasbehälter durch Spülen der oberen Kammer 4 mit dem Restgas überführt.

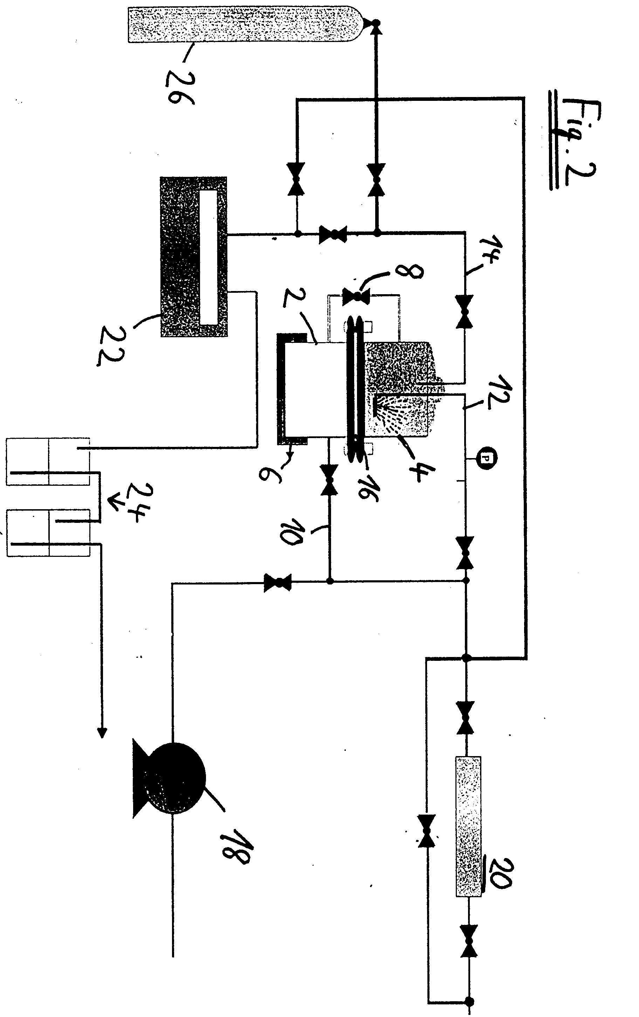

Figur 2 zeigt eine komplette Anlage zur Herstellung des erfindungsgemäßen Gasgemisches, die ebenfalls wieder die Ausgangsstoff-Verdampfungskammer 2 mit Heizung 6, die Reaktorkopfgemischkammer 4, hier mit Sprühdüse für das Restgas, und die Anschlüsse für den Eintrag 10, 12 des Restgases und für den Abzug 14 des Gasgemisches aufweist. Weiterhin sind hier eine Vakuumpumpe 18 vorgesehen, mit der der erforderliche Unterdruck oder die Spülung und Reinigung des Ausgangsstoffes durchgeführt werden können. Ein Gastrockner 20 für die vorhergehende Reinigung des Restgases ist ebenso vorgesehen, wie ein Formaldehyd-Analysator 22, mit dem der Gehalt an Formaldehyd im Ausgangsgasgemisch gemessen werden kann, Gaswaschflaschen 24 und eine Prüfgasflasche 26 zum Befüllen.

## Patentansprüche

1. Verfahren zum Herstellen eines Gasgemisches aus mindestens einer verdampfbaren Substanz, insbesondere eines Prüfgases, in einem Druckgasbehälter durch
- thermolytisches Spalten einer verdampfbaren Substanz in einem Reaktor (Kammer 2)
- Überleiten der so erhaltenen verdampften Substanz in einen getrennten Reaktor (Kammer 4)
- Mischen mit einem Restgas (Gas oder Gasgemisch aus Gasen, die nicht mit der verdampften Substanz reagieren, insbesondere Edelgas oder Stickstoff) in dem zweiten Reaktor (Kammer 4)
- Spülen mit Restgas in den Druckgasbehälter.

2. Verfahren zum Herstellen eines Formaldehyd-Gasgemisches nach Anspruch 1, durch
- thermolytisches Spalten eines Formaldehyd-abgebenden Stoffes wie Paraformaldehyd, Trioxan, Alphapolyoximethylen oder N-Hydroximethylbenzamid in einem Reaktor (Kammer 2)
- Überleiten des so erhaltenen monomeren Formaldehyds in einen getrennten Reaktor (Kammer 4)
- Mischen mit einem Restgas (Gas oder Gasgemisch aus Gasen, die nicht mit Formaldehyd reagieren, insbesondere Edelgas oder Stickstoff, keinesfalls O₂, H₂ oder Wasser enthaltende Gase) in dem zweiten Reaktor (Kammer 4)
- Spülen mit Restgas in den Druckgasbehälter.

3. Verfahren nach Anspruch 1 oder Anspruch 2**, dadurch gekennzeichnet**, daß die thermolytische Spaltung bei 30 bis 400°C, vorzugsweise bei 50 bis 150°C, erfolgt.

4. Verfahren nach einem der vorhergehenden Ansprüche, **dadurch gekennzeichnet**, daß die Überleitung durch einen Differenzdruck erfolgt und die Mischung bevorzugt mit einem Zerstäuber erfolgt.

5. Verfahren nach einem der vorhergehenden Ansprüche, **dadurch gekennzeichnet**, daß das Mischen über einen bestimmten Zeitraum (eine Minute bis ein Tag, bevorzugt eine Minute bis eine Stunde) erfolgt.

6. Verfahren nach einem der vorhergehenden Ansprüche, **dadurch gekennzeichnet**, daß die Überleitung der verdampfbaren Substanz in den zweiten Reaktor (Kammer 4) und die Mischung ein einem Schritt durch Einleiten von Restgas in den ersten Reaktor (Kammer 2) erfolgt.

7. Verfahren nach einem der Ansprüche 1 bis 5, **dadurch gekennzeichnet**, daß die Überleitung von dem ersten Reaktor (Kammer 2) zum zweiten Reaktor (Kammer 4) mittels Diffusion durch eine Membran (16) erfolgt.

8. Verfahren nach einem der vorhergehenden Ansprüche, **dadurch gekennzeich**net, daß das Ausgangsmaterial vorher durch Erwärmen, insbesondere auf Temperaturen zwischen 50 und 75°, Kondensierenlassen und Spülen mit Restgas gereinigt wird.

9. Verwendung des erhaltenen Formaldehyd-Gasgemisches eines der vorhergehenden Ansprüche als Vorgemisch zur Herstellung von Prüfgasen.

10. Verwendung des Formaldehyd-Gasgemisches eines der vorhergehenden Ansprüche als Prüfgas.

11. Formaldehyd-Gasgemisch mit 0,01 ppm (V) bis 10%, insbesondere 0,01 ppm (V) bis 100 ppm (V) oder 0,01 ppm (V) bis 50 % oder 1 ppm (V) bis 5 % Formaldehyd, Rest Restgas wie oben beschrieben, insbesondere nach einem der vorhergehenden Verfahren hergestellt.
